Europäisches Patentamt

European Patent Office

Office européen des brevets

(11) Veröffentlichungsnummer: **0 071 565**
**A1**

# (12) EUROPÄISCHE PATENTANMELDUNG

(21) Anmeldenummer: 82730098.9

(22) Anmeldetag: 22.07.82

(51) Int. Cl.³: **G 01 N 33/54**
**G 01 N 33/56**

(30) Priorität: 25.07.81 DE 3129469

(43) Veröffentlichungstag der Anmeldung:
09.02.83 Patentblatt 83/6

(84) Benannte Vertragsstaaten:
AT BE CH DE FR GB IT LI LU NL SE

(71) Anmelder: SCHERING AKTIENGESELLSCHAFT Berlin
und Bergkamen
Müllerstrasse 170/178 Postfach 65 03 11
D-1000 Berlin 65(DE)

(72) Erfinder: Mettler, Lieselotte, Prof. Dr.
Düsterbrooker Weg 45
D-2300 Kiel 1(DE)

(72) Erfinder: Czuppon, Adam Benedek, Dr.
Charles-Ross-Ring 37
D-2300 Kiel 1(DE)

(54) Verfahren zum Nachweis von Antikörpern gegen Sperma mittels Radio- oder Enzymimmunoassay.

(57) Verfahren zum Nachweis von Antikörpern gegen Sperma, indem man Donorspermatozoen oder aus Spermatozoen gewonnene lösliche Präparationen auf PVC-Microtiterplatten fixiert, die fixierten Spermatozoen mit Testserum inkubiert, anschließend an die fixierten Zellen radioaktiv-markiertes oder Enzym-markiertes Bindungsprotein mit Spezifität für Antikörper, zum Beispiel Protein A, koppelt und die fixierten Radioaktivitäten bzw. Enzymaktivitäten mißt.

Die Erfindung betrifft ein neues Verfahren zum Nachweis von Antikörpern gegen Sperma.

Im Durchschnitt liegt bei 10 - 15 % aller Ehen eine Sterilität vor, die verschiedenste Ursachen haben kann, wie zum Beispiel Veränderungen an den Eileitern, Störungen des ovariellen Zyklus, zervikale Faktoren oder (Auto)antikörperbildung gegen Spermien.

Zur Routineuntersuchung bei der männlichen sowie der weiblichen Sterilität gehören die Bestimmung der Leukozytenzahl im Blut, die Senkungsreaktion der Blutkörperchen, die Luesserologie, die Bestimmung der Blutgruppe und eine Urinsedimentuntersuchung. Wünschenswert wäre auch ein leicht durchzuführender Test auf Autoimmunisierung gegen Sperma.

Es wurden bereits eine Reihe von Nachweismethoden für Sperma-Antikörper vorgeschlagen (A.C. Menge, Techniques of human andrology, North-Holland, Pub. Comp. 1977, S. 225 - 237; A.C. Menge, Immunological aspects of infertility and fertility regulation, Eslvier 1980, S. 205 - 224), wie zum Beispiel der Mikrosperma-Agglutinations-Test (J. Friberg, 1974, Acta Obstet. Gynec. Scand. 36, S. 21 ff), der Mikrosperma-Immobilisations-Test (S. Hustedt, T. Hjort, 1975, Int. J. Fertil. 20, S. 97 ff), der Makroskopische Gelatine-Agglutinations-Test (S. Kibrick et al., 1952, Fertil-Steril 3, S 430 ff) oder der indirekte Immunofluoreszenz-Test (T. Hjort, K.B. Hansen, 1971, Clin. Exp. Imm. 8, S. 9 ff).

Alle diese Tests haben den Nachteil, daß sie schwierig zu interpretieren sind und nur von geschultem Fachpersonal ausgewertet werden können. Darüber hinaus können diese Tests durch unspezifische Reaktionen, zum Beispiel mit Hormonen oder Arzneimitteln negativ beeinflußt werden, so daß diese Tests für Routineuntersuchungen nicht geeignet sind.

Es wurde nun überraschenderweise gefunden, daß man auf einfache und gut reproduzierbare Weise Antikörper gegen Sperma auf folgendem Wege nachweisen kann. Donorspermatozoen oder aus Spermatozoen gewonnene lösliche Präparationen werden auf PVC-Microtiterplatten fixiert und zunächst mit Testserum und anschließend mit radioaktiv markiertem oder Enzym-markiertem Bindungsprotein mit Spezifität für Antikörper, zum Beispiel Protein A, inkubiert. Enthält das Testserum Spermatozoen-Antikörper, so werden diese von den fixierten Spermatozoen oder aus Spermatozoen gewonnenen löslichen Präparationen gebunden. Diese gebundenen Antikörper binden dann ihrerseits das markierte Bindungsprotein, zum Beispiel Protein A, das eine Affinität zum Fc-Teil von Immunglobulinen des IgG-Typs verschiedener Säugetierspezies hat (J. Immunol. Methods 20 (1978), 241). Das gebundene Bindungsprotein, zum Beispiel Protein A, das durch Messung der Radioaktivität oder der Enzymaktivität nach den üblichen Verfahren bestimmt werden kann, ist damit ein Maß für die im Testserum enthaltene Menge an Spermatozoen-Antikörper.

Die Durchführung des Tests geschieht zum Beispiel in folgender Weise: Donorspermatozoen mit einem durchschnittlichen Ejakulatvolumen von 3 - 4 ml mit einer Spermienzahl von 20 - 30 x $10^6$/ml werden mehrmals in PBS (Phosphatgepufferte Salzlösung) bei pH 7,2  10 Minuten bei ca. 4 $^{\circ}$C gewaschen und auf eine endgültige Konzentration von 3 x $10^6$/ml Zellen resuspendiert. Die Zellsuspension wird in 100 µl Portionen in die U-förmigen Wölbungen einer PVC-Microtiterplatte aufgetragen. Die Platten werden zentrifugiert und anschließend abdekantiert. Ca. 100 µl Methanol werden in die einzelnen Aushöhlungen der Microtiterplatten gegeben und anschließend ca. 5 - 15 Minuten zentrifugiert. Nach Entfernung des überflüssigen Methanols läßt man das restliche Methanol verdampfen. Die fixierten Zellen werden mit PBS gewaschen und anschließend 10 - 20 Minuten bei Raumtemperatur in einer PBS-Lösung, welche 0,1 % Gew./Vol. BSA (Rinderserumalbumin) und 0,05 % Vol./Vol. TWEEN$^{(R)}$ 20 enthält, inkubiert. Die Platten können dann bei ca. -20 $^{o}$C so lange aufbewahrt werden, bis sie für den Test gebraucht werden. Bei der Fixierung mit Methanol ist darauf zu achten, daß die Zellen in den U-förmigen Wölbungen der Microtiterplatten einen Monolayer bilden. Bei der Durchführung des Tests wird das Serum in einer Verdünnung von 1:8 mit Barbitalpuffer in die mit Spermatozoen fixierten Aushöhlungen auf PVC-Microtiterplatten ca. 30 Minuten bei Raumtemperatur inkubiert, um nichtspezifische Bindungen zu reduzieren. Danach wird die Platte mehrmals mit Barbitalpuffer gewaschen und 150 µl des Antiserums aufgetragen, 2 Stunden bei Zimmertemperatur inkubiert und einige Zeit bei 4 $^{o}$C stehenge-lassen. Nach nochmaligem Waschen mit Barbitalpuffer werden die gebundenen Antikörper mit radioaktiv markiertem oder Enzym-markiertem Protein A, gelöst in 0,05 M Barbitalpuffer, inkubiert. Nach Abwaschen des ungebundenen Protein A können die fixierten Radioaktivitäten bzw. Enzymaktivitäten nach üblichen Methoden gemessen werden.

Das neue Verfahren hat den Vorteil, mit einer niedrigen Spermenkonzentration auszukommen. So reichen zum Beispiel 2 ml Ejakulat mit einer Spermenzahl von 10 x 10$^{7}$/ml für mehr als 600 Bestimmungen. Die hohe Sensitivität des Tests sowie die einfache Durchführbarkeit ermöglichen es, diesen Test routinemäßig einzusetzen.

Die einzelnen Schritte des Tests sollen durch die folgenden Beispiele näher erläutert werden:

Herstellung der Microtiterplatten

a) <u>mit suspendierten Spermatozoen</u>

Donorspermatozoen mit einem durchschnittlichen Ejakulatvolumen von 3 - 3,5 ml einer Spermenzahl von 20 - 30 x $10^6$/ml werden bei 4 °C 3mal abzentrifugiert (10 Min. x 400 g) und in PBS (4 °C, pH 7,2) gewaschen und dann auf eine endgültige Konzentration von 3 x $10^6$/ml Zellen resuspendiert. Die Zellsuspension wird in 100 µl-Portionen geteilt und in die Aushöhlungen einer PVC-Microtiterplatte (Dynatech Laboratories Inc., Cat. No. 1-220-24 nach der Methode von Stocker und Heusser, J. Imm. Methods, 1979, Bd. 26, S. 87 ff, aufgetragen. Die Platten werden zentrifugiert, 150 g x 10 Minuten bei 4 °C, und der Oberstand abdekantiert. Danach werden ca. 100 µl Methanol in jede Aushöhlung der Microtiterplatte gegeben und weitere 10 Minuten bei 400 g zentrifugiert. Nach Entfernen des Oberstandes läßt man den Rest des Methanols verdampfen. Die fixierten Zellen werden mit PBS gewaschen und 15 Minuten bei Raumtemperatur in PBS-Lösung, welche 0,1 % Gew./Vol. BSA und 0,05 % Vol./Vol. TWEEN$^{(R)}$20 enthält, inkubiert. Die Platten können bei -20 °C bis zum endgültigen Gebrauch aufbewahrt werden.

b) <u>mit gelösten Antigenen</u>

<u>Herstellung von gelösten Antigenen aus Spermatozoen:</u>

Donorspermatozoen werden dreimal mit PBS (Phosphatgepufferter Salzlösung) gewaschen. 5 x $10^8$ Spermatozoen werden in 5 ml 0,3 M Lithium-dijodsalicylat und 1 % Gew./Vol. Triton$^{(R)}$ X-100 in Wasser suspendiert, 10 Minuten mit Ultraschall (Amplitude 2 µ) behandelt und 2 Stunden bei 37 °C inkubiert. Die Suspension wird bei 4 °C 60 Minuten in einer Beckmann Ultrazentrifuge (25.000 Umdrehungen pro Minute) zentrifugiert und die überstehende Schicht 48 Stunden gegen 0,01 M Natrium-Phosphatpuffer (pH 7,2) dialysiert.

### Beschichtung der Platten

Die in einer Konzentration von 7,5 - 10 µg Protein/ml in PBS gelösten Antigene werden in 50 µl-Portionen geteilt und in die Aushöhlungen einer mit Poly(L)-Lysin (100 µg/ml in Wasser) beschichteten PVC-Microtiterplatte aufgetragen. Die mit Antigenen beschichteten Microtiterplatten werden 2 Stunden bei 37 °C inkubiert, mit PBS gewaschen und 30 Minuten bei Raumtemperatur in PBS-Lösung, welche 3 % Gew./Vol. BSA enthält, inkubiert.

### Kontrollseren

Ein Antikörper-enthaltendes Serum und zwei negative Kontrollsera werden eingesetzt. Sie werden ca. 30 Minuten bei 56 °C inkubiert, um das Komplement zu inaktivieren. Als Antiserum wurde Kaninchen-anti-human-Spermatozoen-Antiserum (Behring Institut Marburg, Lot. No. 8401 G) eingesetzt. Der Antikörper-Titer wurde nach dem Mikrosperma-Agglutinations-Test (Hustedt und Hjort 1974) und dem Mikrosperma-Immobilisations-Test (Fridberg 1974) bestimmt. Normales Kaninchenserum und das Antiserum, zweifach adsorbiert mit gewaschenen aber unfixierten menschlichen Spermatozoen ($10^7$ Zellen per 100 µl Serum, 1 Stunde bei 37 °C inkubiert und anschließend 24 Stunden bei 4 °C) dienten als negative Kontrolle.

### Markierung von Protein A

Die Jodierung des Proteins A wurde nach der Methode von Bolton, Biochem. 1973, J. 133, S. 529 ff, durchgeführt. Der Markierungsgrad wurde bestimmt zu 1800 cpm/ng. Für jeden Test wurde eine konstante Menge an $^{125}$J-Protein A verwendet (45.000 cpm in 50 µl einer 0,05 M Barbital-puffer-Lösung bei pH 9,2 - enthaltend 0,1 % Gew./Vol. Gelatine und $5 \times 10^{-5}$ M EDTA).
Enzym-markiertes Protein A (zum Beispiel das Konjugat aus Protein A und alkalischer Phosphatase) ist kommerziell zugänglich (zum Beispiel Conco, Lab.-Division, Begasweg 1, 6200 Wiesbaden, Deutschland).

Immunoassay

Es wurde eine Serie von Zweifachverdünnungen des Testserums sowie des Kontrollserums von 1:16 bis 1:8.192 in 0,05 M Barbitalpuffer bei pH 9,2 hergestellt (von jeder Verdünnungsserie wurden 3 Ansätze hergestellt). Die beschichteten Microtiterplatten werden 30 Minuten bei Raumtemperatur mit 100 μl normalem Kaninchenserum in einer Verdünnung von 1:8 mit Barbitalpuffer inkubiert, um nichtspezifische Bindungen zu entfernen. Nach zweimaliger Waschung der Aushöhlung auf der Microtiterplatte werden 150 μl des Testserums bzw. der Kontrollseren auf die Platte gegeben und 2 Stunden bei Raumtemperatur und anschließend 2 Stunden bei 4 °C inkubiert. Die einzelnen Aushöhlungen der Microtiterplatte werden zweimal mit Barbitalpuffer gewaschen und anschließend werden 50 μl des markierten Protein A, gelöst in einem 0,05 M Barbitalpuffer (pH 9,2 0,1 % Gew./Vol. Gelatine, $5 \times 10^{-5}$ M EDTA), zugegeben. Nach einstündiger Inkubation bei Raumtemperatur und 2 Stunden bei 4 °C werden die Aushöhlungen auf der Platte dreimal mit eiskaltem PBS gespült und ausgestanzt. Bei zum Beispiel [125]J-markiertem Protein A werden die Plastikblöcke im Gammazähler gemessen. Bei zum Beispiel mit alkoholischer Phosphatase markiertem Protein A werden die Plastikblöcke mit p-Nitrophenylphosphat inkubiert und das freigesetzte p-Nitrophenol fotometrisch gemessen. Der Titer des Kaninchen-anti-human-Antiserums wird definiert als die kleinste Verdünnungsstufe, bei welcher ein signifikanter Unterschied in cpm gegenüber dem Kontrollserum (p < 0,05, Student's T-Test) bestimmt werden kann.

Ein Vergleich des erfindungsgemäßen Tests mit dem Microsperma-Agglutinations-Test und dem Microsperma-Immobilisations-Test zeigt seine Überlegenheit.

Die Empfindlichkeit des Microsperma-Agglutinations-Tests für das Kaninchen-anti-humen-Spermatozoen-Antiserum wurde zu 1:64 bestimmt und des Microsperma-Immobilisations-Tests zu 1:32 (Friberg und Hustedt und Hjort-Methode).

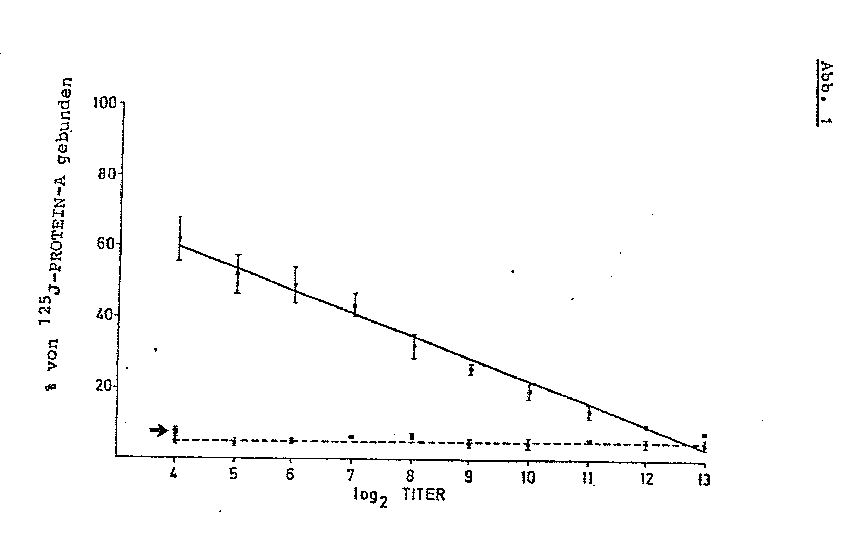

Mit der hier beschriebenen RIA-Methode zum Beispiel wurde jedoch eine Empfindlichkeit von 1:4096 erreicht. Wie aus Abb. 1 bzw. Tab. 1 hervorgeht, war zwischen Meß- und Kontrollwerten bis zur Serumverdünnung 1:8192 ein Unterschied feststellbar. Die Differenz zwischen Meß- und Kontrollwert war bis zur Verdünnung 1:4096 statistisch signifikant ($p < 0{,}05$).

Abbildung 1 zeigt die Menge $^{125}$J-Protein-A gebunden an normales Kaninchenserum und Immunserum. Durchgehende Linie: $^{125}$J-Protein-A, gebunden an Immunserum ($r = 0{,}99$), gestrichelte Linie: $^{125}$J-Protein-A, gebunden an normales Kaninchenserum ($r = 0{,}309$). Der Pfeil zeigt den Prozentsatz an Radioaktivitäten an, die nach Erschöpfung des Testserums mit ungewaschenen Spermatozoen ($10^7$ Zellen / 100 µl Antiserum) noch gebunden wurde. Die gepunktete Linie markiert den Durchschnitt der Dreierbestimmung.

**Tabelle 1:** Vergleich der Mikrosperma-Agglutinations- und Immobilisierungsteste mit dem $^{125}$J-markierten Protein-A verwendenden Immunoassay

| Verdünnung | Kaninchen-anti-human-Spermatozoen-Antiserum | | | normales Kaninchenserum | | |
| --- | --- | --- | --- | --- | --- | --- |
| | Mikrosperma-Test | | Immunoassay | Mikrosperma-Test | | Immunoassay |
| | Aggl. | Immob. | cpm * | Aggl. | Immob. | cpm * |
| 1 : 16 | + | + | 27843 ± 1662 | - | - | 2224 ± 246 |
| 1 : 32 | + | + | 23406 ± 1458 | - | - | 2201 ± 247 |
| 1 : 64 | + | - | 22122 ± 1296 | - | - | 2349 ± 55 |
| 1 : 128 | - | - | 19545 ± 817 | - | - | 2739 ± 13 |
| 1 : 256 | - | - | 14552 ± 904 | - | - | 2925 ± 197 |
| 1 : 512 | - | - | 11556 ± 353 | - | - | 2090 ± 305 |
| 1 : 1024 | - | - | 8725 ± 621 | - | - | 2070 ± 371 |
| 1 : 2048 | - | - | 6136 ± 465 | - | - | 2539 ± 35 |
| 1 : 4096 | - | - | 4390 ± 139 | - | - | 2222 ± 301 |
| 1 : 8192 | - | - | 3626 ± 148 | - | - | 2152 ± 374 |

* Mittelwert aus drei Werten ± (S.E.)

Patentansprüche

1.) Verfahren zum Nachweis von Antikörpern gegen Sperma, dadurch gekennzeichnet, daß man als Antigene Donorspermatozoen oder aus Spermatozoen gewonnene lösliche Präparationen auf PVC-Microtiterplatten fixiert, die fixierten Spermatozoen bzw. Präparationen mit Testserum inkubiert, anschließend an die fixierten Zellen bzw. Präparationen radioaktiv- oder Enzym-markiertes Bindungsprotein mit Spezifität für Antikörper koppelt und die fixierten Radio- bzw. Enzymaktivitäten nach den üblichen Meßverfahren bestimmt.

2.) Verfahren gemäß Anspruch 1, dadurch gekennzeichnet, daß man Donorspermatozoen mit einer Konzentration von $3 \times 10^6$/ml Zellen in Gegenwart von Methanol derart fixiert, daß die Spermatozoen auf den Platten eine Monolayer bilden.

3.) Verfahren gemäß Anspruch 1, dadurch gekennzeichnet, daß man aus Spermatozoen durch Ultraschallbehandlung gewonnene lösliche Präparationen (Antigene) in einer Konzentration von 7,5 - 10 µg Protein/ml mit Poly(L)- Lysin fixiert.

4.) Verfahren gemäß Anspruch 1, dadurch gekennzeichnet, daß als radioaktiv markiertes Bindungsprotein $^{125}$J-markiertes Protein A verwendet wird.

5.) Verfahren gemäß Anspruch 1, dadurch gekennzeichnet, daß als Bindungsprotein Protein A und als Enzym zur Markierung von Protein A alkalische Phosphatase verwendet wird.

6.) Verfahren gemäß Anspruch 1, dadurch gekennzeichnet, daß die fixierten Spermatozoen bzw. Präparationen 30 - 40 Minuten bei Raumtemperatur mit Antikörperfreiem Serum im Verhältnis 1:8 in Gegenwart eines Barbitalpuffers inkubiert werden, anschließend mit Barbitalpuffer gewaschen und dann mit dem Testserum inkubiert werden.

7.) Verfahren gemäß Anspruch 6, dadurch gekennzeichnet, daß als Antikörper-freies Serum normales Kaninchen-serum verwendet wird.

Abb. 1

Europäisches Patentamt

**EUROPÄISCHER RECHERCHENBERICHT**

**0071565**
Nummer der Anmeldung

EP 82730098.9

## EINSCHLÄGIGE DOKUMENTE

| Kategorie | Kennzeichnung des Dokuments mit Angabe, soweit erforderlich, der maßgeblichen Teile | betrifft Anspruch |
|---|---|---|
| D,A | CLINICAL AND EXPERIMENTAL IMMUNOLOGY, Vol. 8, 1971 <br><br> BLACKWELL SCIENTIFIC PUBLICATIONS, Oxford, Edinburgh <br> Seiten 9-23 <br><br> ---- | 1 |

**KLASSIFIKATION DER ANMELDUNG (Int. Cl.³)**

G 01 N 33/54
G 01 N 33/56

**RECHERCHIERTE SACHGEBIETE (Int. Cl.³)**

G 01 N

**KATEGORIE DER GENANNTEN DOKUMENTE**

X: von besonderer Bedeutung allein betrachtet
Y: von besonderer Bedeutung in Verbindung mit einer anderen Veröffentlichung derselben Kategorie
A: technologischer Hintergrund
O: nichtschriftliche Offenbarung
P: Zwischenliteratur
T: der Erfindung zugrunde liegende Theorien oder Grundsätze
E: älteres Patentdokument, das jedoch erst am oder nach dem Anmeldedatum veröffentlicht worden ist
D: in der Anmeldung angeführtes Dokument
L: aus andern Gründen angeführtes Dokument

&: Mitglied der gleichen Patentfamilie, übereinstimmendes Dokument

| X | Der vorliegende Recherchenbericht wurde für alle Patentansprüche erstellt. |
|---|---|

| Recherchenort | Abschlußdatum der Recherche | Prüfer |
|---|---|---|
| WIEN | 28-10-1982 | SCHNASS |

EPA form 1503.1    06.78